# EUROPEAN PATENT SPECIFICATION

(11) **EP 3 241 024 B1**
(45) Date of publication and mention of the grant of the patent: **22.05.2019**
(21) Application number: 15822950.0
(22) Date of filing: 24.12.2015
(51) Int. Cl.: G01N 33/20, C21C 5/46, G01N 1/12

(54) **IMMERSION PROBE AND ASSEMBLY OF IMMERSION SUBLANCE AND IMMERSION PROBE FOR A CONVERTER FURNACE**
TAUCHSONDE UND ANORDNUNG EINER TAUCHSUBLANZE UND TAUCHSONDE FÜR EINEN KONVERTEROFEN
SONDE D'IMMERSION ET ENSEMBLE COMPOSÉ D'UNE SONDE D'IMMERSION ET D'UNE SUB-LANCE D'IMMERSION POUR FOUR CONVERTISSEUR

(30) Priority: 30.12.2014 BR 1433086
(43) Date of publication of application: 08.11.2017
(73) Proprietor: ECIL Met Tec Ltda., 18170-000 Piedade SP (BR); Vesuvius Group, 7011 Ghlin (BE)
(72) Inventor: SASSO, Peterney, 18170-000 Piedade (BR)
(74) Representative: Brohez, Véronique
(86) International application number: PCT/EP2015/081232
(87) International publication number: WO 2016/107821

(56) References cited:
- US-A- 4 566 343
- US-A- 5 789 910
- US-A1- 2007 173 117
- US-B2- 7 370 544

## Description

The present invention relates to an immersion probe and an assembly of immersion sublance and immersion probe to perform measurements and/or take samples out of a converter furnace filled with molten metal, said immersion probe being configured to feature a variable length.

### Background of the Invention

Immersion sublances and immersion probes are broadly used in converter furnaces during steelmaking processes. Typically, pig iron and scrap metal are discharged into the converter furnace, which fuses them together at a high temperature (1600 to 1750°C), thus producing steel.

In order to produce a high quality product showing ideal chemical characteristics, the molten metal has to be submitted to several chemical analyses. The aim is to monitor all the chemical elements contained in the steel, for example by controlling, among others, the carbon, carbon equivalents and silicon concentration, or by monitoring the hardness, resistance, machinability, temperature, oxidation level.

Such chemical analyses are performed by means of sensing or sampling the molten metal, those operations being performed by sensors located at the end of an immersion probe.

Conventionally, during the production of molten metal, a blowing lance is used to blow oxygen inside the converter furnace at supersonic speed onto molten slag and molten metal.

In parallel, the assembly of immersion sublance and immersion probe is inserted from the external environment into the inside of the converter furnace following a longitudinal direction, such that only the immersion probe of said assembly is submerged into molten slag and subsequently into molten metal. Once in contact with molten metal, the immersion probe is able to perform measurements and/or take samples of molten metal by means of sensors or sampling chambers. Upon fulfillment of those tasks, the assembly of immersion sublance and probe is taken out of the converter furnace, and the immersion probe is then disconnected from the immersion sublance and then discarded.

The connection of the immersion probe to the immersion sublance is performed before the insertion of the assembly into the converter furnace, said connection being partially automated.

As previously mentioned, the immersion probe is used only once during the immersion and/or measurement process. On the other hand, the immersion sublance can be reused, for it is provided with a longer service life.

Considering that the immersion sublance is used for several immersions and/or measurements, deformations due to harsh ambient conditions and high temperatures inside the converter furnace have been observed after several immersions and/or measurements by the sublance. Such deficiencies are quite common and severe, resulting in a difficulty to connect a new immersion probe to the immersion sublance.

More specifically, a deformed immersion sublance can present connection problems with a new immersion probe, resulting in clearance between the sublance holder and the immersion probe. Such characteristic is not desirable, as it can lead to various problems.

Since the immersion probe structure is made out of cardboard and does not possess precise dimensions, observation has been made that it cannot completely cover the immersion sublance holder. Furthermore, depending on the climatic conditions as well as the storage location of the immersion probes, it is known that deformations can occur on the cardboard constituting the probes casing, creating radial or longitudinal variations. Considering this problem, it is known that when the assembly of immersion sublance and immersion probe is inserted into the converter furnace, some molten metal splashes might land on the sublance holder, making the connection to a new immersion probe difficult.

In order to go around this problem, an attempt has been made in the art to counter the deformation of the immersion sublance. The European Patent EP-A1-69433 describes a sublance presenting a fixed upper end and a lower end which can rotate. It specifically discloses that the lower portion of the immersion sublance gets deformed after the first immersion in molten metal, then the sublance is taken out of the converter furnace and its lower end is rotated 180°. After said rotation, the sublance is then immersed anew. According to the teachings of European Patent EP-A1-69433, this feature allows the lower portion to come back to its original position (before the first immersions).

Another known solution is described by the US-A-4566343. Said patent discloses an immersion sensor comprising a rubber seal designed to prevent motel metal from penetrating into the immersion sublance.

In addition, a solution aimed at improving the connection between the immersion probe and the sublance holder is also known in the art. US-B2-7370544 describes the use of a spring or an elastic ring on the sublance holder. This solution is problematic, considering that the immersion sublances currently commercialized require some adjustments in order to accommodate the spring or the elastic ring. In this way, it becomes mandatory to stop the measurement process in order to adapt the sublance as previously described.

Considering the above solutions, it is observed that in the state of the art, the use of an immersion probe with a variable connection length, able to compensate for length variations in the longitudinal and/or radial directions of the immersion sublances, is not known.

Furthermore, the current state of the art provides no solution for the use of an immersion probe with a variable connection length without requiring the adaptation of the immersion sublances, such that immersion sublances currently commercialized could be used without requiring any adaptation.

### Aims of the Invention

The first aim of the present invention is to provide an immersion probe enabling a greater connection efficiency with the immersion sublance holder.

The second aim of the present invention is to provide an immersion probe that can be connected to the immersion sublances currently commercialized without any need to adapt them.

Another aim of the present invention is to provide an immersion probe that can be connected to immersion sublances with a variable connection length.

Yet another aim of the present invention is to provide an immersion probe that can be connected to the immersion sublances that have been deformed by consecutive immersions into the converter furnace.

In addition, the present invention also aims at providing an immersion probe that can prevent molten metal from sticking onto the immersion sublance holder.

Providing an immersion probe that prevents connection defects or clearance with the immersion sublance is also among the aims of this invention.

Finally, the present inventions aims at compensating the longitudinal and/or radial length variations of the immersion sublance upon connection.

### Brief Description of the Invention

The aims of the present invention are achieved by means of an assembly of immersion sublance and immersion probe for a converter furnace, the sublance comprising a connecting end, the connecting end being configured so as to be coupled to a first coupling end of a sublance holder, said sublance holder extending longitudinally from the first coupling end to a second coupling end, said sublance holder being connected to the immersion probe by connecting means between the second coupling end of the sublance holder and an adjustable portion of the immersion probe.

The aims of the present invention are also achieved by means of an immersion probe for a converter furnace comprising a casing and an adjustable portion, said casing featuring an internal cavity in order to receive an adjustable portion, said adjustable portion extending longitudinally along the internal cavity of the casing, from a connecting portion to a fixed portion, the connecting portion and fixed portion being associated to one another by means of an elastic portion, the elastic portion being configured so as to change the length of the adjustable portion once a first contact point has been established with the surface of the connecting portion.

### Brief description of the drawings

From now on, the present invention will be more thoroughly described, on the basis of an execution example represented in the drawings. The Figures represent:
Figure 1 - is an inside view of a converter furnace with a blowing lance and an assembly of immersion sublance and immersion probe immersed in motel metal;
Figure 2 - is a representation of the assembly of immersion sublance and immersion probe according to the present invention in a preferred configuration;
Figure 3a - is a representation of a preferred configuration of the immersion sublance when disconnected from the immersion probe;
Figure 3b - is a representation of the immersion sublance when disconnected from the immersion probe, the internal cavity of the sublance holder being illustrated in a preferred configuration;
Figure 4 - is a representation of the immersion probe when disconnected from the sublance in a preferred configuration;
Figures 5a, 5b, 5c, 5d and 5e - are step-by-step representations of how the sublance is connected to an immersion probe of the present invention in a preferred configuration;
Figures 6a, 6b, 6c, 6d, 6e - are step-by-step representations of how the sublance is connected to the immersion probe of the present invention, illustrating in detail the internal cavity of the sublance holder, which is illustrated in a preferred configuration;
Figures 7a, 7b, 7c and 7d - are step-by-step representations of the establishment of a first contact point between the sublance holder and the immersion probe of the present invention in a preferred configuration;

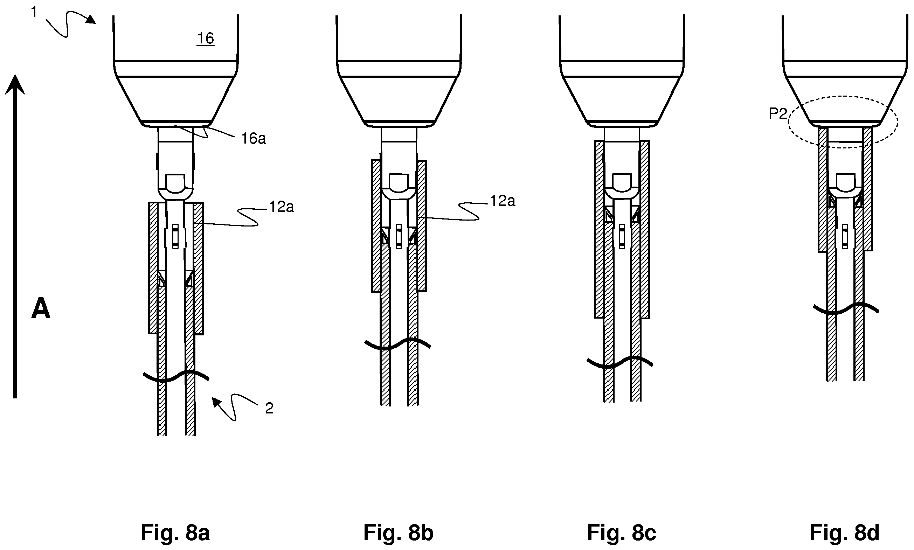

Figures 8a, 8b, 8c and 8d - are step-by-step representations of the establishment of a second contact point between the sublance holder and the immersion probe of the present invention in a preferred configuration;
Figure 9 - is a representation of the sensing head and its components of the present invention in a preferred configuration.
Figure 10 - is a representation of a second embodiment of the present invention, and
Figure 11 - is a representation of a third embodiment of the present invention.

### Detailed description of the figures

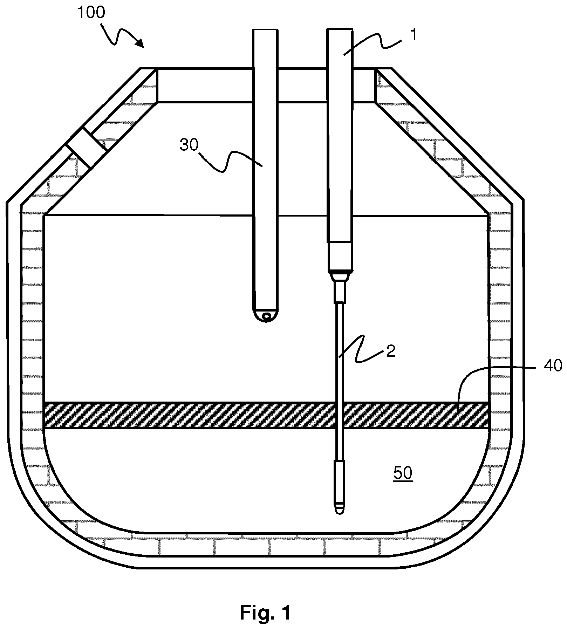

Figure 1 - illustrates an inside view of a converter furnace 100 used in steel industry for producing and refining steel. During the production of molten metal, a blowing lance 30 is used inside the converter furnace 100, said blowing lance 30 being used for injecting oxygen at supersonic speed on molten slag 40 and molten metal 50.

Still in reference to Figure 1, an assembly formed by an immersion sublance 1 connected to an immersion probe 2 can be observed, said assembly formed by said components being inserted into the inside of a converter furnace 100. The assembly of immersion sublance 1 and immersion probe 2 being configured so as to project itself longitudinally from the external environment into the direction of the inside of the converter furnace 100. More specifically, such projection is realized so that the immersion probe 2 is immersed into the molten metal 50 lying underneath the molten slag 40. When immersed, the immersion probe 2 performs measurements and/or takes samples of molten metal 50. Afterwards, it is disconnected from the sublance 1 and then discarded.

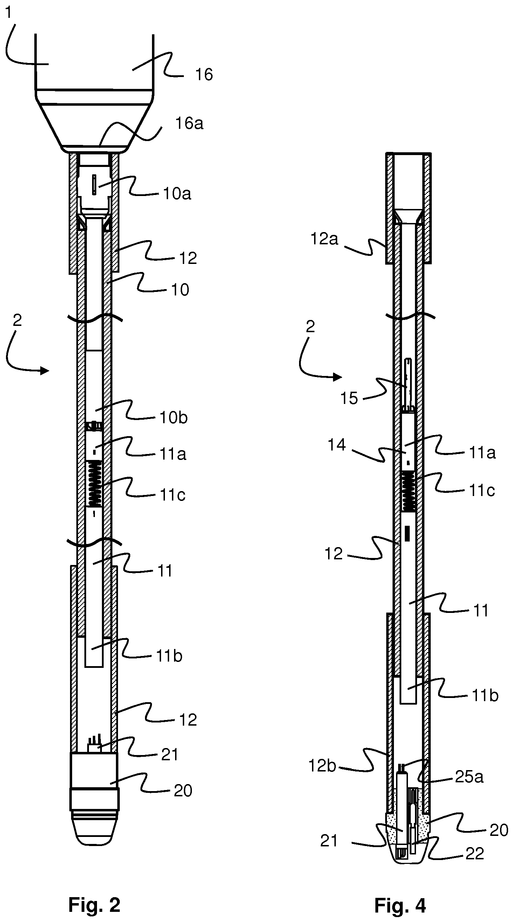

From Figure 2, an assembly of immersion sublance 1 and immersion probe 2 can be observed in a preferred configuration of the present invention, the connection between both elements constituting said assembly being shown in details.

It is observed that if the immersion sublance 1 is used for measurement of molten metal 50, this is realized by means of sensors 21 located at the end of the immersion probe 2 to be immersed in the molten metal 50. Generally, the sensors 21 are connected to a measuring instrument (not shown), located in the surrounding environment of the converter furnace 100. The connection between the sensors 21 and the measuring instrument (not shown) being realized by contact lines that run through the inside of the immersion probe 2 and immersion sublance 1 up to the external environment. After the measurement, the immersion probe 2 is disconnected from the sublance 1 and then discarded.

In case the immersion sublance 1 is used to take molten metal 50 samples, it is observed that this is realized by means of a chamber 22 located at the end of the immersion probe 2 to be immersed in the molten metal 50. After the sampling, the molten metal 50 that has been collected is removed for further analysis, the immersion probe 2 being disconnected from the sublance 1 and then discarded.

Alternatively, the immersion sublance 1 can simultaneously perform measuring and sampling of the molten metal 50, being characterized by the presence of a chamber 22 and sensors 21 as previously described.

In order to provide a better understanding of the present invention, the immersion sublance 1 and the immersion probe 2 are hereunder described separately. The connection of both elements to form the assembly of immersion sublance 1 and immersion probe 2 will be detailed later on.

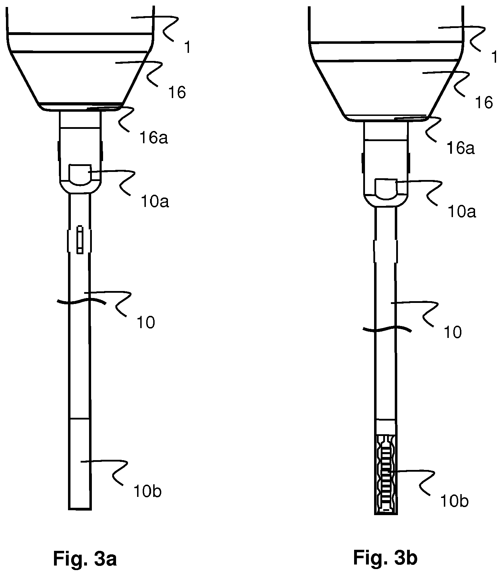

In a preferred configuration, an immersion sublance 1 can be observed in details from Figure 3.

Figures 3a and 3b show the mechanical structure of the immersion sublance 1, the sublance 1 being provided with a guide 16 and a sublance holder 10.

In a preferred configuration of the present invention, the guide 16 presents a substantially cylindrical/tubular shape, being hollow along its whole length. Such a configuration provides the creation of an internal cavity for the passage of sensors 21 contact lines up to the surrounding environment and subsequently to a measuring instrument (not shown).

The aforementioned guide 16 is preferably made from metallic materials able to withstand the typical high temperatures (1600 to 1750°C) of a converter furnace 100.

Still in reference to Figures 3a and 3b and in a preferred configuration, it can be observed that the guide 16, on its portion that is closest to the converter furnace 100, features an end that will from now on be referred to as the guide connecting end 16a. As can be observed, the guide connecting end 16a is coupled to the sublance holder 10 of the sublance 1.

Preferably, the sublance holder 10 features a substantially cylindrical/tubular shape, said sublance holder 10 extending longitudinally from a first coupling end 10a in direction of the bottom of the converter furnace 100, down to a second coupling end 10b.

It can be observed that the first coupling end 10a is coupled to the guide connecting end 16a, such that the diameter of the guide connecting end 16a is equal to the external diameter of the first coupling end 10a, thus presenting a perfect coupling.

Regarding the second coupling end 10b, as will be described later, it must present an external diameter similar to the one of the internal cavity of the immersion probe 2.

From Figure 3b, it is observed that in a preferred configuration, the internal cavity of the immersion sublance 1 located at the coupling end 10b. Furthermore, it can be noticed that in a preferred configuration, said cavity can feature at least one holder contact line 25c, at least one of the lines 25c being arranged circumferentially around the inner wall of the cavity of the holder 10 coupling extremity 10b. At least one line 25c is connected to the measuring instrument located in the surrounding environment of the converter furnace 100.

The immersion sublance 1 having been described, from now on the immersion probe 2 will be described. In a preferred configuration, the immersion probe 2 can be observed in details from Figure 4.

Figure 4 shows the mechanical structure of the immersion probe 2, said probe comprising a casing 12, an adjustable portion 11 and a sensing head 20.

Preferably, the immersion sublance 2 casing 12 is made from cardboard and can feature different lengths or diameters, these being dimensioned according to the user.

The casing 12 is designed with several layers of cardboard, such that the casing 12 of the immersion probe 2 does not completely disintegrate when the immersion probe 2 is immersed into molten metal 50. Considering that the immersion lasts a few seconds, the structure of the casing 12 formed of several layers of cardboard is able to withstand the typical high temperatures (1600 to 1700°C) of a converter furnace 100.

In a preferred configuration, the casing 12 features a substantially cylindrical/tubular shape along its whole length, such a configuration allowing the creation of an internal cavity to receive the adjustable portion 11, the internal diameter being wide enough to receive the above referred to portion 11.

Furthermore, it can also be observed from Figure 4 that, in a preferred configuration, said casing 12 comprises a casing upper portion 12a and a casing lower portion 12b. Considering that both portions 12a and 12b feature the same diameter, it is observed that an intermediate portion of the casing 12 features a smaller diameter.

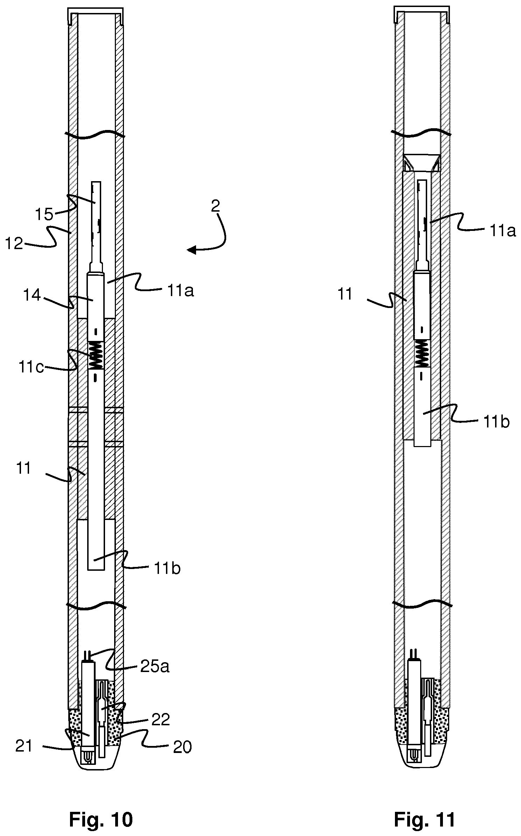

Alternatively, as observed from Figures 11 and 12, the casing 12 can feature a constant diameter along its whole length.

In respect of the adjustable portion 11, it is observed from Figure 4 that it extends longitudinally along the inside of the internal cavity of the casing 12 from a connecting portion 11a to a fixed portion 11b. The connecting portion 11a being close to the casing upper portion 12a and the fixed portion 11b being close to the casing lower portion 12b. The connecting portion 11a and the fixed portion 11b being associated to one another by means of an elastic portion 11c, such that the adjustable portion 11 features a variable length.

In a preferred configuration, it is observed that the connecting portion 11a and the fixed portion 11b feature a substantially cylindrical/tubular shape comprising diameters smaller or equal to the internal cavity of the casing 12.

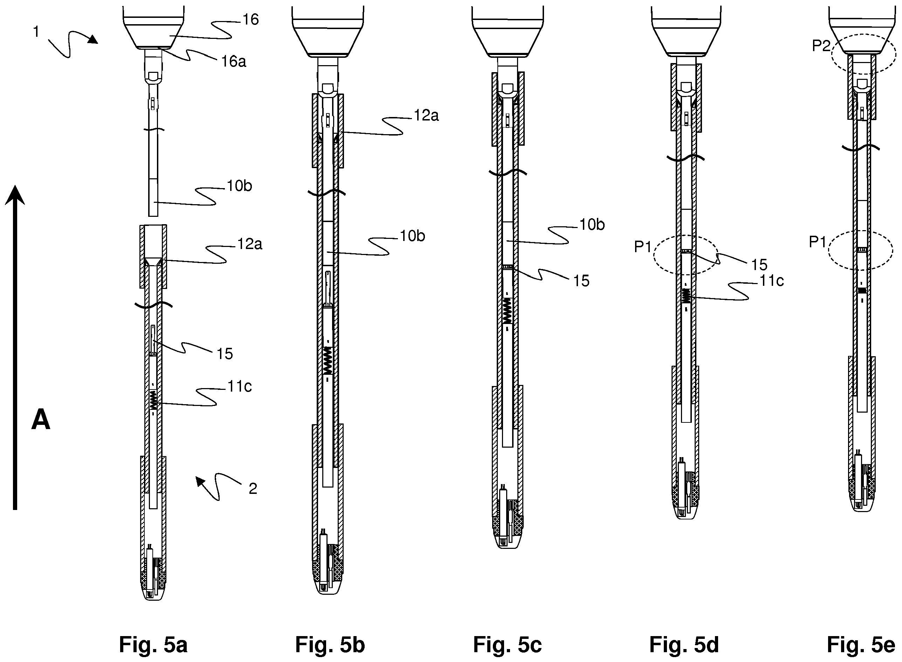

In respect of the elastic portion 11c, it can be a spring, an elastic ring, an elastomer, or any other elastic material, able to compress and expand itself by means of applying a force once a first contact point P1 is established such as observed from Figures 5a-5e, 6a-6e and 7a-7e and that will be subsequently described in details.

In respect of the connecting portion 11a, it is observed from Figure 4 that it comprises a connection base 14 and a connector 15. The connection base 14 being located at the end of the connecting portion 11a that is closest to the bottom of the converter furnace 100. The connector 15 being located at the end of the connecting portion 11a that is closest to the top of the converter furnace 100. The connection base 14 being associated to the connector 15e and the elastic portion 11c. The Connector 15 projecting in the direction of the top of the converter furnace 100.

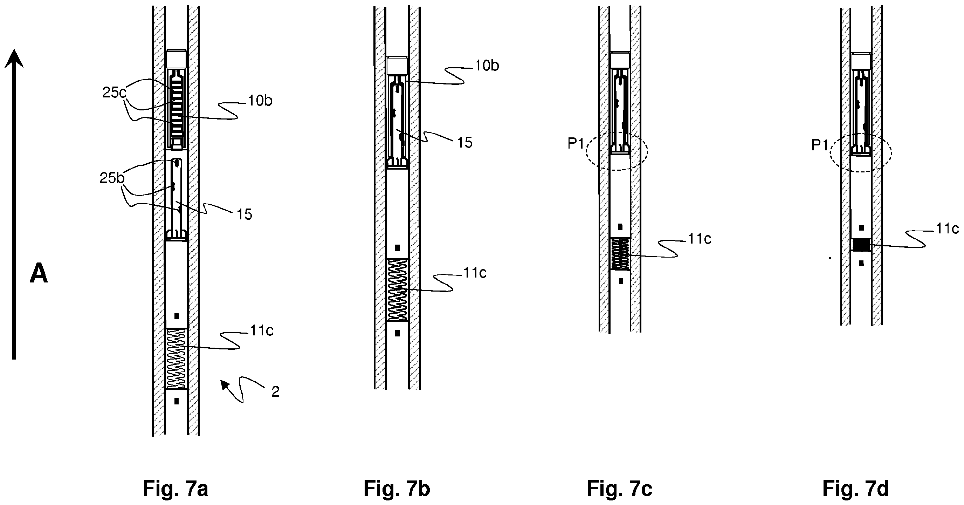

In a preferred configuration, it can be observed from Figures 7a to 7e that the connector 15 is provided with at least one connector contact line 25b, at least one line 25b being circumferentially located around the surface of the connector 15. As will be described later, at least one contact line of the connector 25b will be connected to at least one contact line of the holder 25c.

Preferably, the connector 15 dimensions are such that it presents a substantially smaller diameter than the base 14, the connector 15 presenting a diameter smaller or equal to the diameter of the second coupling end 10b. The base 14 in turn presenting a diameter smaller or equal to the one of the second coupling end 10b. Such configuration provides a perfect connection between the immersion sublance 1 and the immersion probe 2, as will be further described.

In respect to the fixed portion 11b, as can be observed from Figure 4, it projects itself towards the bottom of the converter furnace 100, the fixed portion 11b being coupled to the casing lower portion 12b. It is observed that the sensing head 20 is also coupled to the casing lower portion 12b.

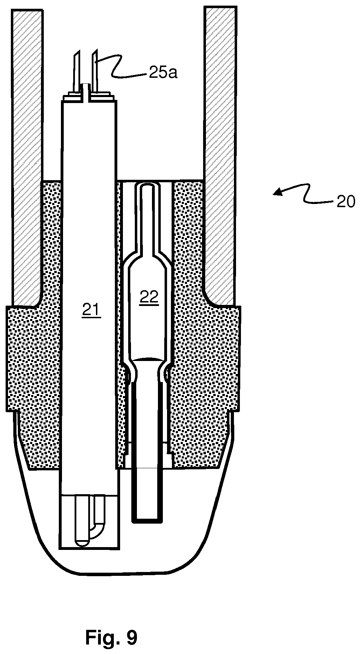

In a preferred but not compulsory configuration, it can observed from Figure 9 that the sensing head 20 is provided with at least one sensor 21, that could either be a temperature sensor, an oxygen sensor, or any other sensor required for molten metal chemical analyses 50. At least one sensor 21 is provided with at least one sensor contact line 25a, the line 25a extending up to the connecting portion 11a, where is it then connected to at least one connector contact line 25b.

Additionally, the sensing head 20 can comprise a sampling chamber 22, said chamber being configured to collect molten metal 50 when the immersion probe is immersed, the collected molten metal 50 solidifying when the immersion probe 2 is taken out of the converter furnace 100.

The immersion sublance 1 and immersion probe 2 having been described, from now on the description will focus on the realization of the connection between the immersion sublance 1 and the immersion probe 2, such connection resulting in an assembly of immersion sublance 1 and immersion probe 2.

During steelmaking processes in a converter furnace 100, the user must connect a new immersion probe 2 to the immersion sublance 1, this constituting an assembly of immersion sublance 1 and immersion probe 2.

From Figures 5a to 5e and 6a to 6a, said connection can be observed step-by-step, the main focus being only the guiding end 16 of the immersion sublance 1.

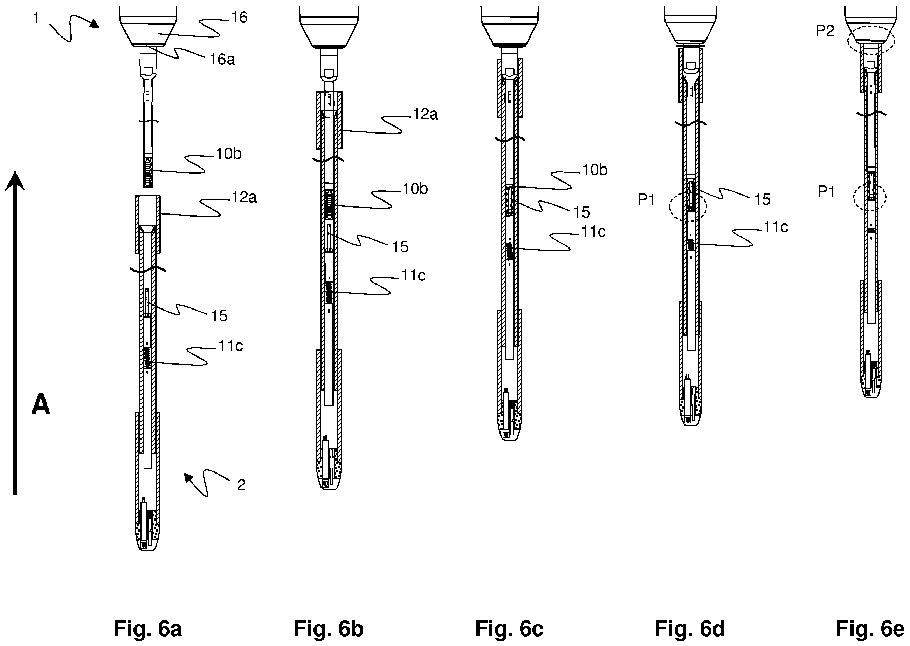

Figures 5a and 6a illustrate a case where the immersion sublance 1 and the immersion probe 2 are completely disconnected. It is observed that an arrow A represents the direction of connection realized by the user for said connection of the elements.

Once the immersion probe 2 has been moved in the direction of connection A, the sublance holder 10 of the immersion sublance 1 is connected to the immersion probe 2. More specifically, the second coupling end 10b is projected into the internal cavity of the immersion sublance 2 towards the adjustable portion 11, as observed from Figure 5b. As previously noted, the external diameter of the second coupling end 10b is smaller or equal to the one of the internal cavity of the immersion probe 2.

It is thus observed that the casing 12 of the immersion probe 2 will encase the sublance holder 10 and the adjustable portion 11. Once the second coupling end 10b is inserted into the internal cavity of the immersion sublance 2, the second coupling end 10b projects itself towards the connector 15 of the connecting portion 11a.

Considering that the connector 15 features a diameter equal or smaller than the one of the internal diameter of the second coupling end 10b, said connector will project itself towards the internal cavity of the second coupling end 10b.

As can be observed from Figures 5b, 5c, and 5d, the connector 15 will project itself until the surface of the second coupling end 10b establishes a first contact point P1 with the surface of the connection base 14 of the connecting portion 11a.

Such first contact point is established by the connection base 14 of the connecting portion 11a which presents a diameter equal to the external diameter of the second coupling end 10b.

Still considering the movement of the immersion probe 2 in the direction of connection A and a first contact point P1 having been established, it is observed that the elastic portion 11c will be compressed, its length thus varying.

Furthermore, from Figure 7a a detailed view of the connection between the connector 15 and the coupling end 10b can be observed, as well as the connector contact lines 25b and the holder contact lines 25c. To be projected towards the internal cavity of the coupling end 10b, the contact lines of the connector 25b connect themselves to the contact lines of the holder 25c. Such connection can be observed from Figures 7b and 7c. Still, it can be noted from figures 7b and 7c that a perfect connection between the lines 25b and 25c is realized when the first contact point P1) is established.

Preferably, the elastic portion 11c is compressed until the casing upper portion 12a is connected to the guide connecting end 16a, such as observed on Figure 5e. It is observed that when these are connected, a second contact point P2 will be established.

It is thus observed that a first and a second contact points P1, P2 are established in the connection between the immersion sublance 1 and the immersion probe 2, such contact points P1, P2 establishing a perfect connection to form the assembly of immersion sublance 1 and immersion probe 2. The assembly presenting a perfect electrical connection by connecting means between the contact lines 25b and 25c and perfect mechanical connection by connecting means between the casing upper portion 12a and the guide connecting end 16a.

It can also be observed that after the connection of the immersion sublance 1 to the immersion probe 2, the assembly is ready to be used in the converter furnace 100. It is observed that, because of this being used in the inside of a converter furnace 100, the immersion sublance 1 can suffer deformations such as previously described.

As previously mentioned, the sublance holder 10, when exposed to high temperatures 1600 to 1750°C and successive immersions, can suffer deformations, presenting radial or longitudinal variations.

Such longitudinal or radial variations are compensated by the elastic portion 11c, the exchange of the immersion sublance 1 thus not being required, making it useable hundreds of times. It is observed that the adjustable portion 11 features a length that varies with the length of the sublance holder 10 of the sublance 1.

The elastic portion 11c features a spring tension effect that is superior to the force required by the connector 15 on the second coupling end 10b. Furthermore, the elastic portion 11c features a "spring effect tension" inferior to the force required for the connection of the adjustable portion 11 of the immersion probe 2 to the sublance holder 10 of the sublance 1.

It is observed that even if the sublance holder 10 of the immersion sublance 1 was slightly deformed, the connection of the connector 15 to the second coupling end 10b could be accomplished because the elastic portion 11c allows the connector 15 to move along the longitudinal direction of the immersion probe 2.

Furthermore, the compensation of longitudinal or radial variations accomplished by the elastic portion 11c allows the user to use immersion sublances 1 that feature variations in connection length. In addition, considering that the casing 12 made out of cardboard can present longitudinal or radial variations depending on the climatic conditions, as well as the storage location of the immersion probes, it is observed that the elastic portion 11c will also compensate such variations.

As an example of preferred realization has been described, it should be understood that the scope of the present invention includes other possible variations, which are only limited by the claims.

## Claims

1. Assembly of **immersion sublance (1)** and **immersion probe (2)** for a converter furnace (100), said assembly being **characterized in that**
- the **sublance (1)** comprises a guide (16) and a guide connecting end (16a), said guide connecting end (16a) being configured to be coupled to a first coupling end (10a) of a **sublance holder** (10), said sublance holder (10) extending longitudinally from the first coupling end (10a) to a second coupling end (10b),
- the **immersion probe (2)** comprises an adjustable portion (11) and a casing (12) having an upper portion (12a), said casing (12) featuring an internal cavity in order to receive an adjustable portion (11), said adjustable portion (11) extending longitudinally along the internal cavity of the casing (12) from a connecting portion (11a) to a fixed portion (11b), said connecting portion (11a) and said fixed portion (11b) being associated to one another by means of an **elastic portion (11c),**
**and wherein**
said sublance holder (10) being connected to the immersion probe (2) by connecting means between the second coupling end (10b) of the sublance holder (10) and the connecting portion (11a) of the adjustable portion (11) of the immersion probe (2) said connection portion (11a) presenting a connection base (14) and a connector (15), said connection base being associated to the elastic portion (11c), and the connector (15) being associated to the connection base,
- **the elastic portion** (11c) is configured so as to change the length of the adjustable portion (11) once a first contact point (P1) has been established between the surface of the connecting base (14) of the connecting portion (11a) and the second coupling end surface (10b), and
the elastic portion (11c) is compressed until the casing upper portion (12a) is connected to the guide connecting end (16a) establishing a second contact point P2.

2. Assembly of immersion sublance (1) and immersion probe (2) for a converter furnace (100) according to claim 1, **characterized in that** the adjustable portion
(11) extends longitudinally and features a length that varies with the length of the sublance holder (10).

3. Assembly of immersion sublance (1) and immersion probe (2) for a converter furnace (100) according to claim 1, **characterized in that** the elastic portion (11c) is a spring, an elastic ring, an elastomer or any other elastic material.

4. Assembly of immersion sublance (1) and immersion probe (2) for a converter furnace (100) according to claim 1, **characterized in that** the sublance (1) and the immersion probe (2) are substantially tubular.

5. Assembly of immersion sublance (1) and immersion probe (2) for a converter furnace (100) according to claim 1, **characterized in that** the diameter of the connection base (14) is equal to the outside diameter of the second coupling end (10b) and the diameter of the connector (15) is
smaller or equal to the diameter of the second coupling end (10b).

6. Assembly of immersion sublance (1) and immersion probe (2) for a converter furnace (100) according to claim 1, **characterized in that** when the sublance holder (10) is connected to the immersion probe (2), the connector (15) of the connecting portion (11a) extends along the internal cavity of the second coupling end (10b) until a first contact point (P1) is established between the second coupling end (10b) and the surface of the connection base (14) of the connecting portion (11a).

7. Assembly of immersion sublance (1) and immersion probe (2) for a converter furnace (100) according to claim 1, **characterized in that** the adjustable portion (11) of the immersion probe (2) comprises a sensing head (20).

8. Assembly of immersion sublance (1) and immersion probe (2) for a converter furnace (100) according to claim 7, **characterized in that** the sensing head (20) comprises one or more of the following features: a temperature sensor, an oxygen sensor or a sampling chamber (22).

9. Assembly of immersion sublance (1) and immersion probe (2) for a converter furnace (100) according to claim 8, **characterized in that** at least one sensor contact line (25a) of at least one sensor (21) is guided from the sensing head (20) to a measuring instrument.

## Patentansprüche

1. Anordnung einer Tauch-Sublanze (1) und einer Tauchsonde (2) für einen Konverterofen (100), wobei die Anordnung **dadurch gekennzeichnet ist, dass**
- die Sublanze (1) eine Führung (16) sowie ein Führungs-Verbindungsende (16a) umfasst, wobei das Führungs-Verbindungsende (16a) so konfiguriert ist, dass es mit einem ersten Kupplungs-Ende (10a) einer Sublanzen-Halterung (10) gekuppelt werden kann, wobei die Sublanzen-Halterung (10) in Längsrichtung vom ersten Kupplungs-Ende (10a) zu einem zweiten Kupplungs-Ende (10b) verläuft,
- die Tauchsonde (2) einen einstellbaren Bereich (11) sowie ein Gehäuse (12) umfasst, das einen oberen Bereich (12a) besitzt,
wobei das Gehäuse (12) einen inneren Hohlraum aufweist, um einen einstellbaren Bereich (11) aufzunehmen,
wobei der einstellbare Bereich (11) in Längsrichtung entlang des inneren Hohlraums des Gehäuses (12) von einem Verbindungs-Bereich (11a) zu einem fixierten Bereich (11b) verläuft, wobei der Verbindungs-Bereich (11a) und der fixierte Bereich (11b) einander über einen elastischen Bereich (11c) zugeordnet sind,
und wobei
die Sublanzen-Halterung (10) mit der Tauchsonde (2) mithilfe einer Verbindungs-Einrichtung zwischen dem zweiten Kupplungs-Ende (10b) der Sublanzen-Halterung (10) und dem Verbindungs-Bereich (11a) des einstellbaren Bereichs (11) der Tauchsonde (2) verbunden ist,
wobei der Verbindungs-Bereich (11a) eine Verbindungs-Basis (14) sowie einen Anschlussteil (15) aufweist,
wobei die Verbindungs-Basis dem elastischen Bereich (11c) zugeordnet ist und der Anschlussteil (15) der Verbindungs-Basis zugeordnet ist,
wobei der elastische Bereich (11c) so konfiguriert ist, dass die Länge des einstellbaren Bereichs (11) verändert wird, wenn einmal ein erster Kontakt-Punkt (P1) zwischen der Oberfläche der Verbindungs-Basis (14) des Verbindungs-Bereichs (11a) und der Oberfläche des zweiten Kupplungs-Endes (10b) errichtet wurde, und
der elastische Bereich (11c) komprimiert wird, bis der obere Gehäusebereich (12a) mit dem Führungs-Verbindungsende (16a) verbunden ist, wodurch ein zweiter Kontaktpunkt P2 errichtet wird.

2. Anordnung einer Tauch-Sublanze (1) und einer Tauchsonde (2) für einen Konverterofen (100) gemäß Anspruch 1, **dadurch gekennzeichnet, dass** der einstellbare Bereich (11) in Längsrichtung verläuft und eine Länge aufweist, die sich mit der Länge der Sublanzen-Halterung (10) ändert.

3. Anordnung einer Tauch-Sublanze (1) und einer Tauchsonde (2) für einen Konverterofen (100) gemäß Anspruch 1, **dadurch gekennzeichnet, dass** der elastische Bereich (11c) aus einer Feder, aus einem elastischen Ring, aus einem Elastomer oder aus irgendeinem anderen elastischen Material besteht.

4. Anordnung einer Tauch-Sublanze (1) und einer Tauchsonde (2) für einen Konverterofen (100) gemäß Anspruch 1, **dadurch gekennzeichnet, dass** die Sublanze (1) und die Tauchsonde (2) im Wesentlichen rohrförmig ausgebildet sind.

5. Anordnung einer Tauch-Sublanze (1) und einer Tauchsonde (2) für einen Konverterofen (100) gemäß Anspruch 1, **dadurch gekennzeichnet, dass** der Durchmesser der Verbindungs-Basis (14) gleich dem Außendurchmesser des zweiten Kupplungs-Endes (10b) ist, und dass der Durchmesser des Anschlussteils (15) kleiner als oder gleich dem Durchmesser des zweiten Kupplungs-Endes (10b) ist.

6. Anordnung einer Tauch-Sublanze (1) und einer Tauchsonde (2) für einen Konverterofen (100) gemäß Anspruch 1, **dadurch gekennzeichnet, dass** der Anschlussteil (15) des Verbindungs-Bereichs (11a) dann, wenn die Sublanzen-Halterung (10) mit der Tauchsonde (2) verbunden wird, entlang des inneren Hohlraums des zweiten Kupplungs-Endes (10b) verläuft, bis zwischen dem zweiten Kupplungs-Ende (10b) und der Oberfläche der Verbinddungs-Basis (14) des Verbindungs-Bereichs (11a) ein erster Kontaktpunkt (P1) errichtet ist.

7. Anordnung einer Tauch-Sublanze (1) und einer Tauchsonde (2) für einen Konverterofen (100) gemäß Anspruch 1, **dadurch gekennzeichnet, dass** der einstellbare Bereich (11) der Tauchsonde (2) einen Sensor-Kopf (20) umfasst.

8. Anordnung einer Tauch-Sublanze (1) und einer Tauchsonde (2) für einen Konverterofen (100) gemäß Anspruch 7, **dadurch gekennzeichnet, dass** der Sensor-Kopf (20) eines oder mehrere der folgenden Merkmale umfasst: einen Temperatur-Sensor, einen Sauerstoff-Sensor oder eine Probenentnahme-Kammer (22).

9. Anordnung einer Tauch-Sublanze (1) und einer Tauchsonde (2) für einen Konverterofen (100) gemäß Anspruch 8, **dadurch gekennzeichnet, dass** zumindest eine Sensor-Verbindungsleitung (25a) von zumindest einem Sensor (21) vom Sensor-Kopf (20) zu einem Messinstrument geführt wird.

## Revendications

1. Assemblage d'une sub-lance d'immersion (1) et d'une sonde d'immersion (2) pour un four convertisseur (100), ledit assemblage étant **caractérisé en ce que**
- la sub-lance (1) comprend un guide (16) et une extrémité de connexion de guide (16a), ladite extrémité de connexion de guide (16a) étant configurée pour être couplée à une première extrémité d'accouplement (10a) d'un support de sub-lance (10), ledit support de sub-lance (10) s'étendant longitudinalement depuis la première extrémité de couplage (10a) vers une seconde extrémité d'accouplement (10b),
- la sonde d'immersion (2) comprend une partie réglable (11) et un boîtier (12) ayant une partie supérieure (12a),
ledit boîtier (12) comportant une cavité interne afin de recevoir une partie réglable (11),
ladite partie réglable (11) s'étendant longitudinalement le long de la cavité interne du boîtier (12) depuis une partie de connexion (11a) jusqu'à une partie fixe (11b), ladite partie de connexion (11a) et ladite partie fixe (11b) étant associées l'une à l'autre au moyen d'une partie élastique (11c),
et où
ledit support de sub-lance (10) est relié à la sonde d'immersion (2) par des moyens de connexion entre la deuxième extrémité de couplage (10b) du support de sub-lance (10) et la partie de connexion (11a) de la partie réglable (11) de la sonde d'immersion (2) ladite partie de connexion (11a) présente une base de connexion (14) et un connecteur (15), ladite base de connexion étant associée à la partie élastique (11c), et le connecteur (15) étant associé à la base de connexion,
- la partie élastique (11c) est configurée de manière à modifier la longueur de la partie réglable (11) une fois qu'un premier point de contact (P1) a été établi entre la surface de la base de connexion (14) de la partie de connexion (11a) et la seconde surface d'extrémité de couplage (10b), et
la partie élastique (11c) est comprimée jusqu'à ce que la partie supérieure du boîtier (12a) soit connectée à l'extrémité de connexion de guidage (16a) établissant un second point de contact P2.

2. Assemblage d'une sub-lance d'immersion (1) et d'une sonde d'immersion (2) pour un four à convertisseur (100) selon la revendication 1, **caractérisé en ce que** la partie réglable (11) s'étend longitudinalement et présente une longueur qui varie avec la longueur du porte sub-lance (10).

3. Assemblage d'une sub-lance d'immersion (1) et d'une sonde d'immersion (2) pour un four de convertisseur (100) selon la revendication 1, **caractérisé en ce que** la partie élastique (11c) est un ressort, une bague élastique, un élastomère ou tout autre matériau élastique.

4. Assemblage d'une sub-lance d'immersion (1) et d'une sonde d'immersion (2) pour un four de convertisseur (100) selon la revendication 1, **caractérisé en ce que** la sub-lance (1) et la sonde d'immersion (2) sont essentiellement tubulaires.

5. Assemblage d'une sub-lance d'immersion (1) et d'une sonde d'immersion (2) pour un four à convertisseur (100) selon la revendication 1, **caractérisé en ce que** le diamètre de la base de connexion (14) est égal au diamètre extérieur de la seconde extrémité de couplage (10b) et le diamètre du connecteur (15) est inférieur ou égal au diamètre de la seconde extrémité de couplage (10b).

6. Assemblage d'une sub-lance d'immersion (1) et d'une sonde d'immersion (2) pour un four à convertisseur (100) selon la revendication 1, **caractérisé en ce que** lorsque le support de sub-lance (10) est relié à la sonde d'immersion (2), le connecteur (15) de la partie de connexion (11a) s'étend le long de la cavité interne de la deuxième extrémité de liaison (10b) jusqu'à ce qu'un premier contact (P1) soit établi entre la deuxième extrémité de liaison (10b) et la surface de la base de connexion (14) de la partie de connexion (11a).

7. Assemblage d'une sub-lance d'immersion (1) et d'une sonde d'immersion (2) pour un four de convertisseur (100) selon la revendication 1, **caractérisé en ce que** la partie réglable (11) de la sonde d'immersion (2) comprend une tête de détection (20).

8. Assemblage d'une sub-lance d'immersion (1) et d'une sonde d'immersion (2) pour un four à convertisseur (100) selon la revendication 7, **caractérisé en ce que** la tête de mesure (20) présente une ou plusieurs des caractéristiques suivantes : un capteur de température, un capteur d'oxygène ou une chambre de prélèvement (22).

9. Assemblage d'une sub-lance d'immersion (1) et d'une sonde d'immersion (2) pour un four à convertisseur (100) selon la revendication 8, **caractérisé en ce que** au moins une ligne de contact de capteur (25a) d'au moins un capteur (21) est transmise par la tête de détection (20) à un dispositif de mesure.
